(19) Europäisches Patentamt
European Patent Office
Office européen des brevets

(11) Publication number: **0 000 149**
**A1**

# (12) EUROPEAN PATENT APPLICATION

(21) Application number: **78100163.1**

(51) Int. Cl.$^2$: **C 07 D 239/82**

(22) Date of filing: **15.06.78**

(30) Priority: **16.06.77 US 807076**

(43) Date of publication of application: **10.01.79 Bulletin 79/1**

(84) Designated Contracting States: **BE CH DE FR GB LU NL SE**

(71) Applicant: **E.I. du Pont de Nemours and Company, 1007 Market Street, Wilmington, Del. 19898 (US)**

(72) Inventor: **Middleton, William Joseph, RD 2, Box 62 A Ridge Road, Chadds Ford Pennsylvania 19317 (US)**

(74) Representative: **von Kreisler, Alek, Dipl.-Chem. et al, Deichmannhaus am Hauptbahnhof, D-5000 Köln 1 (DE)**

## (54) Process for preparing quinazoline oxides.

(57) Process for the preparation of quinazolinone-oxides of formula

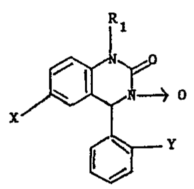

where: X = Cl, Br, $NO_2$, $CF_3$
Y = H, Br, Cl, F
$R_1$ = H, $CH_3$

Such compounds are useful as intermediates in the preparation of 3-fluorobenzodiazepines, which are useful as tranquilizers, muscle relaxants and sedatives.

EP 0 000 149 A1

Patentanwälte
Dr.-Ing. Schön... Dr.-Ing. Th. Meyer Dr.-Ing. Eishold
Dr. Fues Dipl. Chem. Alek von Kreisler
Dipl.-Chem. C... ...keller · Dipl.-Ing. Selting
5 Köln 1, Deichmannhaus





## BACKGROUND OF THE INVENTION

Copending U.S. Patent Application Serial Number 687,318, filed May 26, 1976 by Elena M. Bingham and William Joseph Middleton, which is a continuation-in-part of U.S. Patent Application Serial Number 597,502, now abandoned, discloses certain novel 3-fluorobenzodiazepines of the formula:

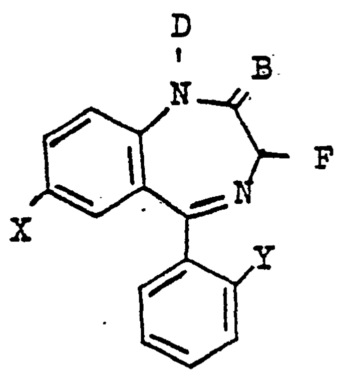

where

X is Cl, Br, $NO_2$ or $CF_3$;

Y is H, Cl, Br or F;

D is H, hydrocarbyl of 1-4 carbons, $-CH_2CF_3$, -CONHR, $-CH_2CH_2NR_2$, or $-CH_2CH_2NR_2 \bullet A$, where R is alkyl of 1-4 carbons and A is a pharmaceutically suitable acid;

B is O; or

0000149

B and D together is =N-N=C(R')-

where R' is H or $C_1$-$C_4$ alkyl,

and the use of such compounds as tranquilizers, muscle relaxants and sedatives in mammals. In addition, Bingham and Middleton disclose a process for making such compounds by reaction of the corresponding 3-hydroxybenzodiazepine with a dialkylaminosulfur trifluoride as follows:

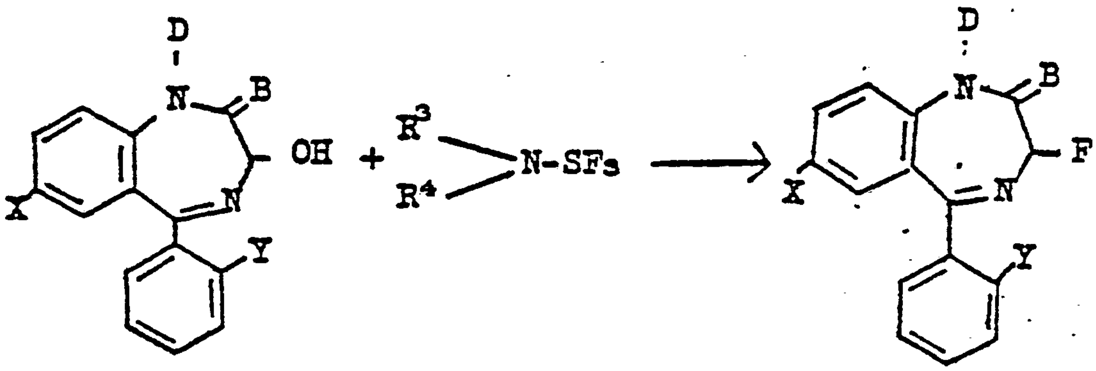

where $R^3$ and $R^4$ are a primary alkyl group of 1-4 carbons or taken together are $-(CH_2)_4-$ or $-(CH_2)_5$.

Copending U.S. Patent Application Serial Number , filed simultaneously with the present application by William Joseph Middleton (attorney' docket number CR-7796) discloses an improved process for preparing such 3-fluorobenzodiazepines, which improved process can be summarized schematically by the following equations:

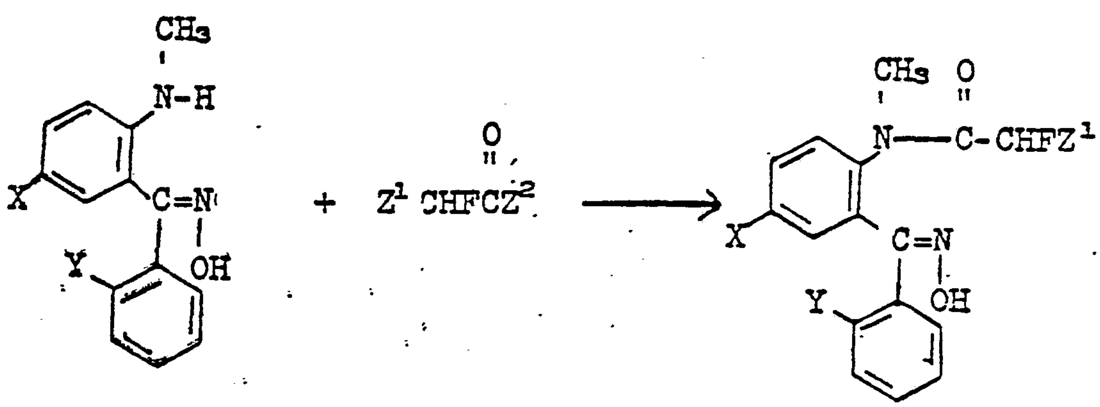

(a) (b) (c)

(c)

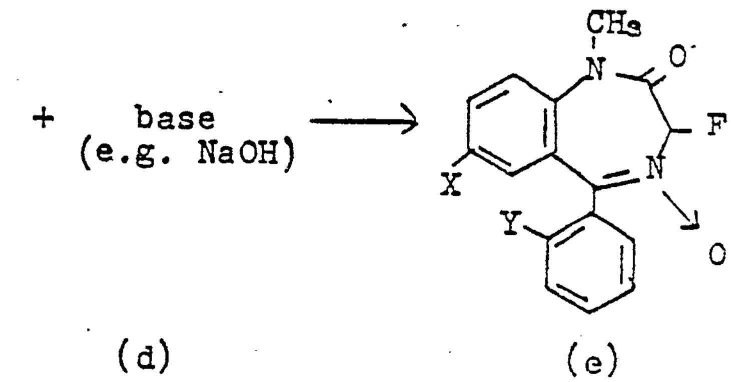


(d) (e)

(e)

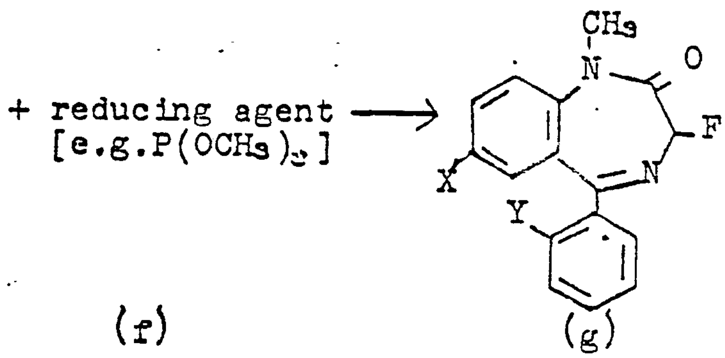


(f) (g)

where

X is Cl, Br, $No_2$ or $CF_3$;
Y is H, Cl, Br or F; and
$Z^1$ and $Z^2$ are Cl or Br.

Middleton also discloses that starting material (1) can be prepared by the process disclosed in U.S. Patent No. 3,398,139.

In addition, copending U.S. Patent Application Serial No. , filed simultaneously herewith by Elena M. Bingham and Arthur J. Elliott (attorney's docket number CR-7795) discloses an improved process for preparing the N-methylaminobenzophenone <u>anti</u>-oxime used as the starting material in Middleton's improved process discussed immediately above. The Bingham and Elliott process can be summarized schematically by the following equations:

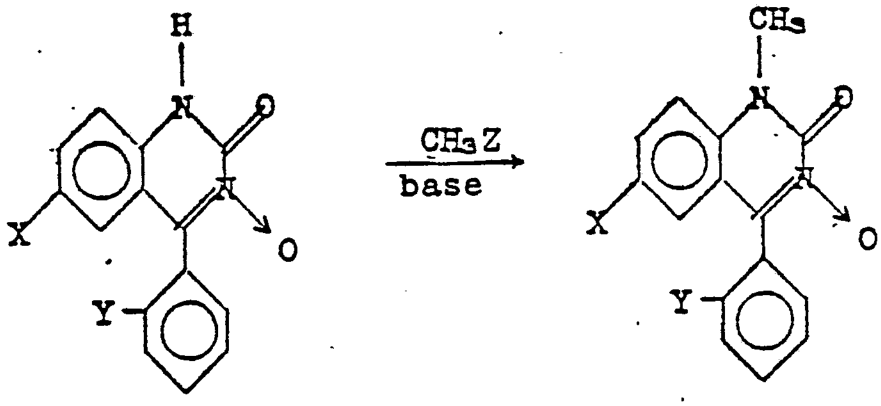


(1) (2) (3)

(3)

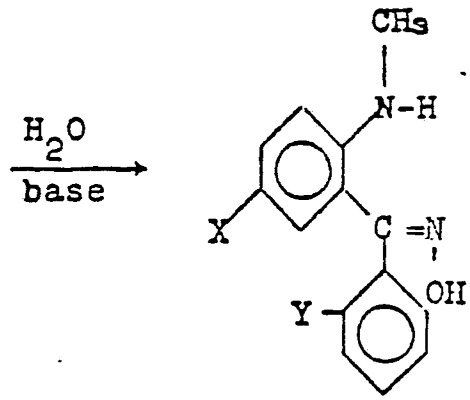


(4) (5)

where

X is Cl, Br, $NO_2$ or $CF_3$;
Y is H, Cl, Br or F; and
Z is I, Br, $CF_3SO_2O$-, $FSO_2O$, $CCl_3SO_2O$- or $CH_3OSO_2O$-.

Bingham and Elliott also disclose that the starting material quinazolinone 3-oxides can be prepared by a process taught by Sulkowski and Childress, J. Org. Chem., 27, 4424 (1962).

SUMMARY OF THE INVENTION

The present invention relates to an improved process for making the quinazolinone 3-oxides of formula (1), above, and an alternate process for making the quinazolinone 3-oxides of formula (3), above.

0000149

More specifically, the present invention relates to an improved process for preparing 6-substituted-4-phenylquinazolinone 3-oxides by the treatment of 2-aminobenzophenone-isocyanate reaction products with hydroxylamine salts.

Organic isocyanates of formula RNCO (where R is hydrocarbyl or halohydrocarbyl of 1-8 carbon atoms) react with 2-aminobenzophenones of formula I (X = Cl, Br, $CF_3$ or $NO_2$; Y = H, Br, Cl or F; $R^1$ = H or $CH_3$) to give either ureas of formula II or quinazolinones of formula III (X, Y and $R^1$ as previously defined), depending on the reaction conditions and the isocyanates used.

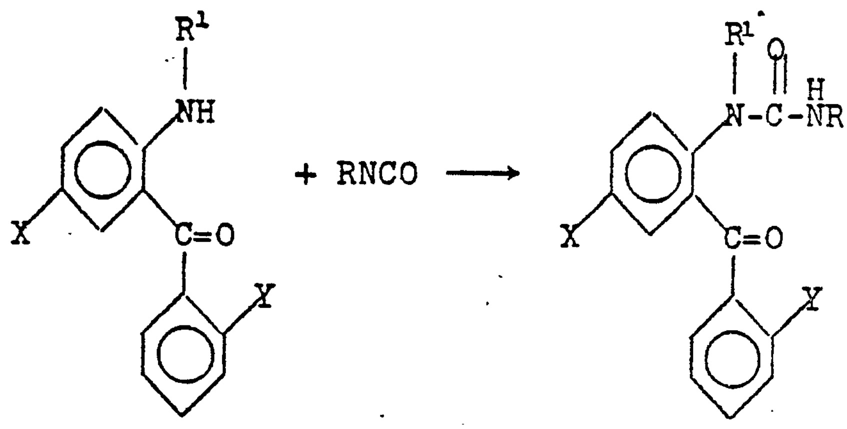


and/or

I

II

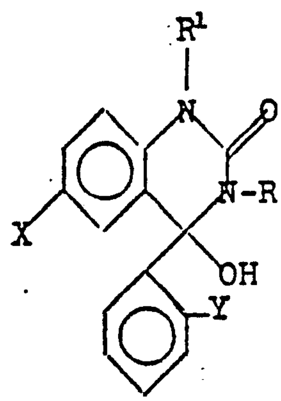

III

These reaction products (either or both II and III) can then be converted to quinazolinone oxides of formula IV by treating them with an acid salt of hydroxylamine.

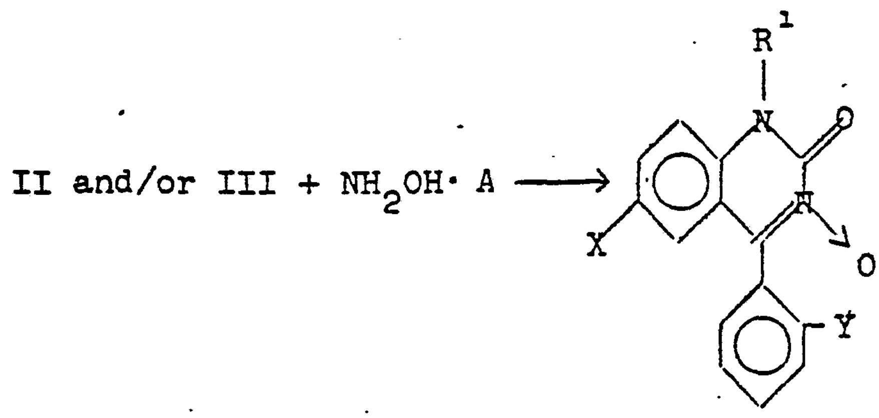


where A is an organic or inorganic acid with a pKa of less than 2.

## DETAILED DESCRIPTION OF THE INVENTION

### Process Conditions

Quinazolinone oxides of formula IV can be prepared by heating a solution or mixture of 2-aminobenzophenoneisocyanate reaction products (of Formula II or III) and an acid addition salt of hydroxylamine in an alcohol solvent. The reaction is conveniently carried out at the reflux temperature of the alcohol solvent, but temperatures from 40° to 200°C are operable. Alcohol solvents useful for this reaction include, but are not limited to, ethanol, methanol, propanol, isopropanol, butanol, 2-methoxyethanol, ethylene glycol, and propylene glycol. Salts of hydroxylamine useful in this reaction include salts with organic or inorganic acids having a pKa of less than 2, such as hydroxylamine hydrochloride, hydroxylamine hydrobromide and hydroxylamine sulfate. The time required for the reaction varies from a few minutes when more reactive isocyanate adducts or higher boiling alcohols are used, to a few days or even weeks when less reactive isocyanate

adducts or lower boiling alcohols are used.

The product quinazolinone oxides can be isolated from the reaction mixture by conventional means. In most cases, the quinazolinone oxides are considerably less soluble than the reactants, and will precipitate during the course of the reaction. When this occurs, the product quinazolinone oxides can be isolated by simply filtering the reaction mixture.

The 2-aminobenzophenone-isocyanate adducts used as starting materials can be prepared by the reaction of 2-aminobenzophenones with organic isocyanates as illustrated in the following Examples or as described by Sulkowski et al. J. Org. Chem., 27 4424 (1962) or by Metlesics et al., J. Org. Chem., 31 1007 (1966).

Alternatively, the compounds of formula IV can be prepared by heating a compound of formula II in a suitable alcohol followed by treatment with a suitable acid addition salt of hydroxylamine.

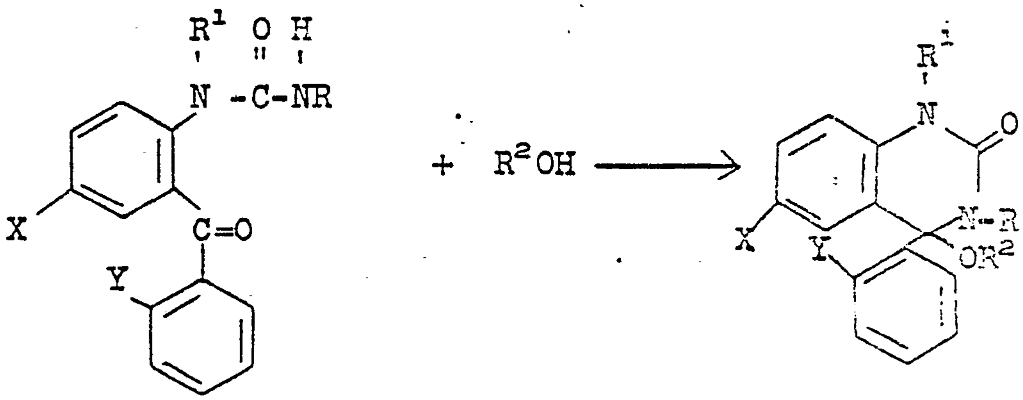


(II) (V)

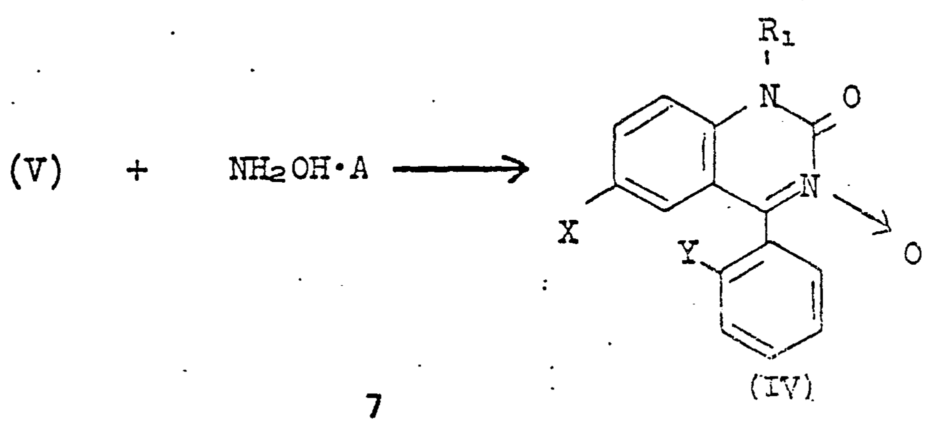


(IV)

where $R^2OH$ is lower aliphatic alcohol of 1-6 carbon atoms, preferably methanol or ethanol.

EXAMPLE 1

Part A. 6-Chloro-3,4-dihydro-4-hydroxy-3-methyl-4-phenyl-2(1H)-quinazolinone

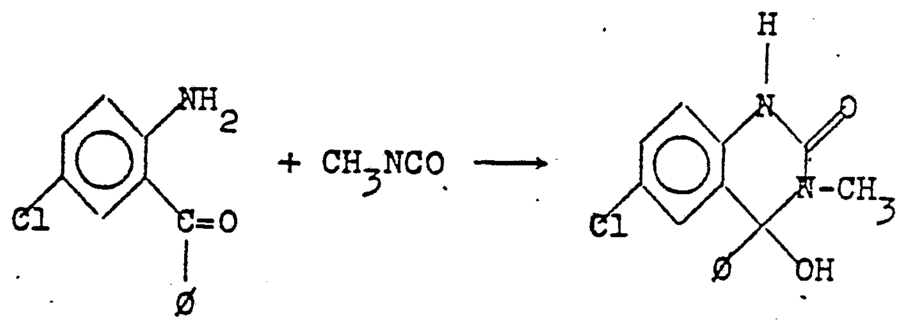

A solution of 100 g (0.43 mole) of 2-amino-5-chlorobenzophenone and 40 g (0.7 mole) of methyl isocyanate in 300 ml of methylene chloride was refluxed for two days and then cooled. The solid portion of the reaction mixture was collected on a filter and washed with methylene chloride to give 119.8 g (96% yield) of 6-chloro-3,4-dihydro-4-hydroxy-3-methyl-4-phenyl-2(1H)-quinazolinone as a white crystalline powder: mp 296-298° (dec); $^1H$ nmr (DMSO-$d_6$), δ 2.66 ppm (s, 3H), 6.8-7.6 ppm (m, 8H) and 10.0 ppm (s, NH); $^{13}C$ nmr (DMSO-$d_6$) δ 86.6 ppm (for COH) and 151.4 ppm (for NHCO).

Anal. Calcd for $C_{15}H_{13}ClN_2O_2$: C, 62.39; H, 4.54; N, 9.70
Found: C, 62.10; H, 4.67; N, 9.53

Part B. 6-Chloro-4-phenyl-2(1H)-quinazolinone 3-Oxide

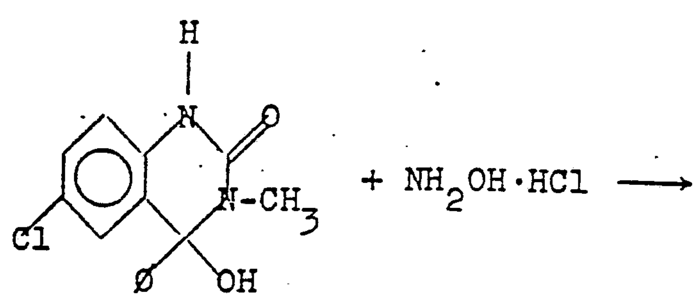

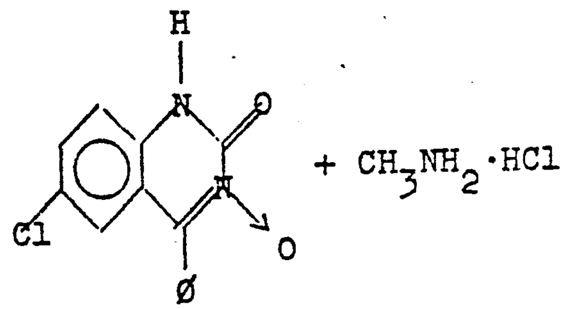


A stirred mixture of 86.6 g (0.3 mole) of 6-chloro-3,4-dihydro-4-hydroxy-3-methyl-4-phenyl-2(1H)quinazolinone and 62.5 g (0.9 mole) of hydroxylamine hydrochloride in 1500 ml ethanol was refluxed for 187 hours, and then cooled. The solid portion of the reaction mixture was collected on a filter, washed with ethanol, and dried in air to give 67.9 g (83%) of 6-chloro-4-phenyl-2(1H)-quinazolinone 3-oxide as yellow crystals: m.p. 267-269°.

EXAMPLE 2

Part A. 6-Chloro-3-ethyl-3,4-dihydro-4-hydroxy-4-phenyl-2(1H)quinazolinone

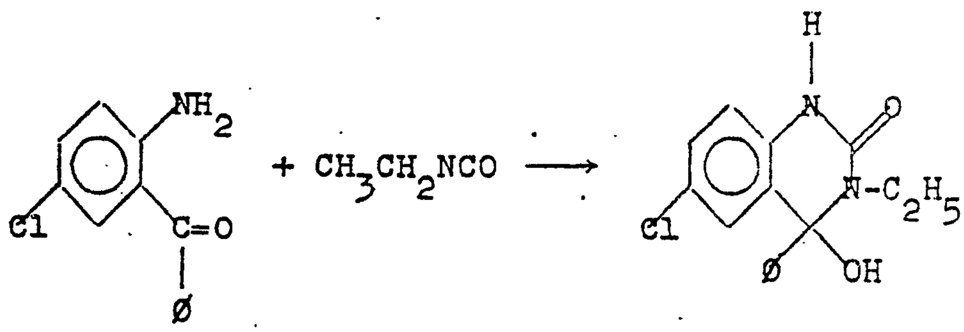


A solution of 28.4 g (31.7 ml, 0.4 mole) of ethyl isocyanate and 46.3 g (0.2 mole) of 2-amino-5-chlorobenzophenone in 100 ml of methylene chloride was refluxed for 20 hours. The reaction mixture was cooled, and the solid portion was collected on a filter and washed with methylene chloride to give 50.72 g (84%) of 6-chloro-3-ethyl-3,4-dihydro-4-hydroxy-4-phenyl-2(1H)quinazolinone as colorless crystals: m.p. 182-184°; $^{13}C$ nmr (DMSO-$d_6$) δ 86.9 ppm (for COH) and δ 151.0 ppm (for NHCO).

<u>Anal</u>. Calc'd. for $C_{16}H_{15}ClN_2O_2$: C, 63.47; H, 4.99; N, 9.25

Found: C, 63.29; H, 4.83, N, 9.46

<u>Part B. 6-Chloro-4-phenyl-2(1H)quinazolinone 3-Oxide</u>

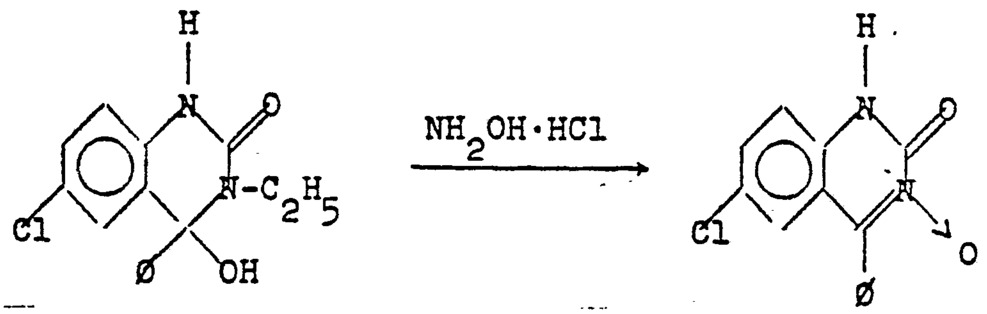


A stirred mixture of 12.11 g (0.04 mole) of 6-chloro 3-ethyl-3,4-dihydro-4-hydroxy-4-phenyl-2(1H)quinazolinone and 8.34 g (0.12 mole) of hydroxylamine hydrochloride in 200 ml ethanol was refluxed for 3 days and then cooled. The solid portion of the reaction mixture was collected on a filter, washed with ethanol, and dried to give 9.27 g (85%) of 6-chloro-4-phenyl-2(1H)quinazolinone 3-oxide as yellow crystals, m.p. 267-269°.

EXAMPLE 3

<u>Part A. 6-Chloro-3,4-dihydro-4-hydroxy-1,3-dimethyl-4-phenyl-2(1H)quinazolinone</u>

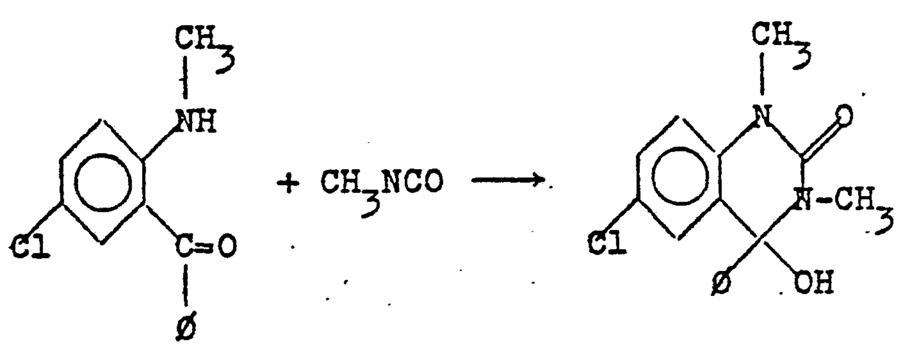

A solution of 12.3 g (0.05 mole) of 5-chloro-2-methylaminobenzophenone and 6 ml (0.1 mole) of methyl isocyanate in 50 ml of methylene chloride was refluxed for 3 days, and then cooled. The solid portion of the reaction mixture was collected on a filter and washed with methylene chloride to give 7.05 g (47%) of 6-chloro-3,4-dihydro-4-hydroxy-1,3-dimethyl-4-phenyl-2(1H)-quinazolinone as light yellow crystals; m.p. 174-175°; $^1$H nmr (DMSO-$d_6$) δ 2.67 ppm (s, 3H), 3.38 ppm (s, 3H), 6.8-7.7 ppm (m, 9H).

Anal. Calc'd. for $C_{16}H_{15}ClN_2O_2$: C, 63.47; H, 4.99; N, 9.25

Found: C, 63.44; H, 4.96; N, 8.84

Part B. 6-Chloro-1-methyl-4-phenyl-2(1H)quinazolinone 3-Oxide

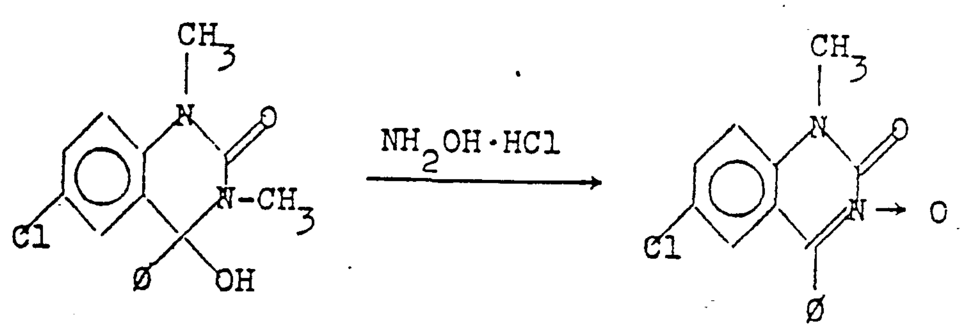


A stirred mixture of 3.03 g (0.01 mole) of 6-chloro-3,4-dihydro-4-hydroxy-1,3-dimethyl-4-phenyl-2(1H)quinazolinone and 2.09 g (0.03 mole) of hydroxylamine hydrochloride in 50 ml of ethanol was refluxed for 5 days. The reaction mixture was cooled, and the solid portion was collected on a filter, washed with ethanol, and dried in air to give 1.75 g (61%) of 6-chloro-1-methyl-4-phenyl-2(1H)quinazolinone 3-oxide as yellow crystals: m.p. 289-291°; $^1$H nmr (TFA) δ 4.22 ppm (s, 3H) and 7.6-8.5 ppm (m, 8H).

EXAMPLE 4

Part A. 1-(2-Benzoyl-4-chlorophenyl)-3-isopropylurea

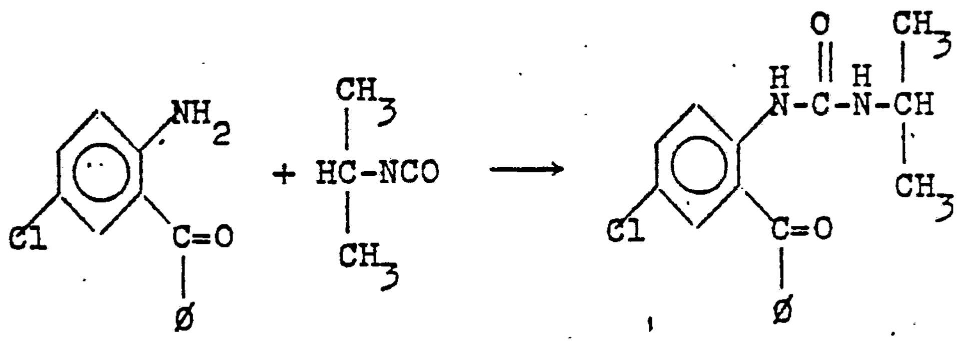

A mixture of 14 g (0.06 mole) of 2-amino-5-chlorobenzophenone and 40 ml of isopropyl isocyanate was refluxed for 3 hours. The solid that formed was suspended in 25 ml of hexane, and then collected on a filter and recrystallized from ethanol to give 12.0 g (63%) of 1-(2-benzoyl-4-chlorophenyl)-3-isopropylurea as colorless needles: m.p. 190-192°; $^1$H nmr ($CDCl_3$) $\delta$ 1.19 ppm (d, J = 6 Hz, 6H), 3.98 ppm (m, 1H), 4.95 ppm (m, NH), 7.2-7.8 ppm (m, 7H), 8.5 ppm (d, J = 10 Hz, 1H) and 10.1 ppm (NH); $^{13}$C nmr (DMSO-$d_6$) $\delta$ 195.4 ppm (C=O) and 153.9 ppm (NHCO).

Anal. Calc'd. for $C_{17}H_{17}ClN_2O_2$: C, 64.45; H, 5.41; N, 8.85

Found: C, 64.21; H, 5.40; N, 8.78

Part B. 6-Chloro-4-phenyl-2(1H)quinazolinone 3-Oxide

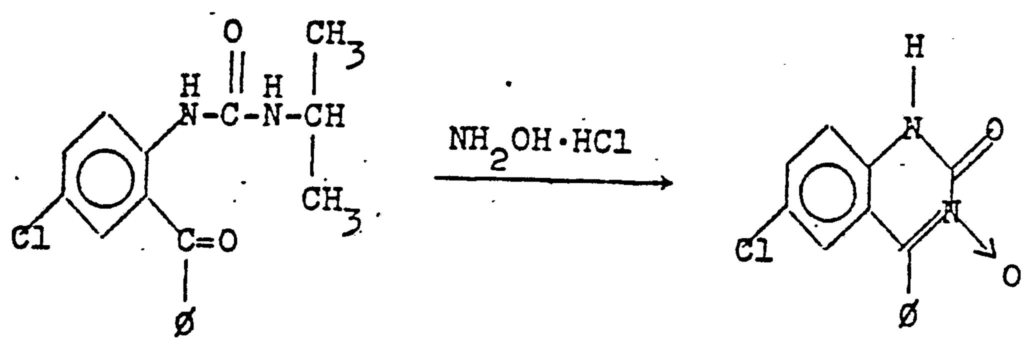

A stirred mixture of 6.34 g (0.02 mole) of 1-(2-benzoyl-4-chlorophenyl)-3-isopropylurea and 4.17 g (0.06 mole) of hydroxylamine hydrochloride in 100 ml of ethanol was refluxed for 48 hours, and then cooled. The suspended crystals were collected on a filter, washed with alcohol, and then dried in air to give 4.60 g (84%) of 6-chloro-4-phenyl-2(1H)quinazolinone 3-oxide as yellow crystals, m.p. 267-269° (dec.).

EXAMPLE 5

Part A. 1-(2-Benzoyl-4-chlorophenyl)-3-phenylurea

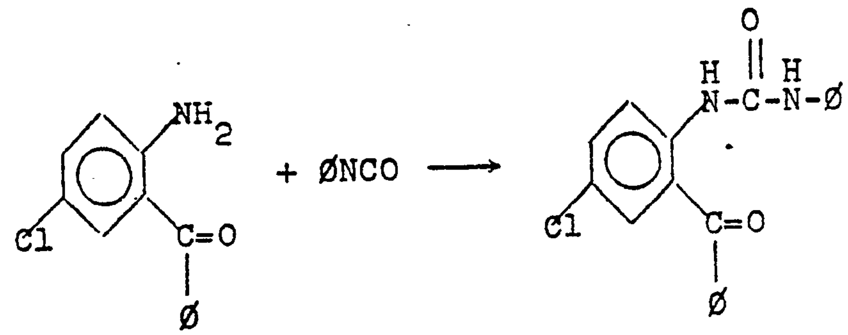

A solution of 13.1 g (0.11 mole) of phenyl isocyanate and 23.17 g (0.1 mole) of 2-amino-5-chlorobenzophenone in 70 ml of methylene chloride was refluxed for 20 hours, and then evaporated to dryness under reduced pressure. The residue was recrystallized from ethanol to give 31.71 g (90%) of 1-(2-benzoyl-4-chlorophenyl)-3-phenylurea as colorless crystals: m.p. 145-147°; $^1H$ nmr (DMSO-$d_6$) δ 6.7-8.3 ppm (m, 13H), 9.43 ppm (d, J = 7 Hz, 1H, ex$D_2O$) and 10.25 ppm (s, 1H, ex$D_2O$); $^{13}C$ nmr (DMSO-$d_6$) δ 152.2 ppm (NHCO) and 195.5 ppm (C=O).

Anal. Calc'd. for $C_{20}H_{15}ClN_2O_2$: C, 68.21; H, 4.50; N, 8.02

Found: C, 68.21; H, 4.50; N, 8.02

Part B. 6-Chloro-4-phenyl-2(1H)quinazolinone 3-Oxide

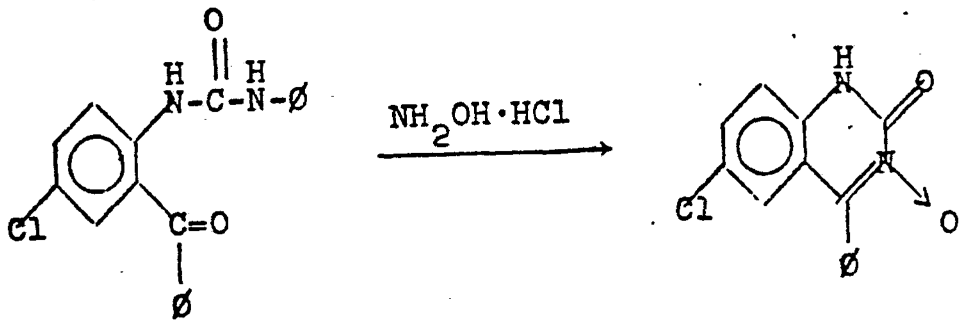


A stirred mixture of 7.02 g (0.02 mole) of 1-(2-benzoyl-4-chlorophenyl)-3-phenylruea and 4.17 g (0.06 mole) of hydroxylamine hydrochloride in 100 ml of ethanol was refluxed for 22 hours, and then cooled. The suspended solid was collected on a filter, washed with ethanol, and dried in air to give 3.82 g (70%) of 6-chloro-4-phenyl-2)1H)quinazolinone as yellow crystals; m.p. 267-269°.

EXAMPLE 6

Part A. 1-(2-Benzoyl-4-chlorophenyl)-1-methyl-3-phenylurea

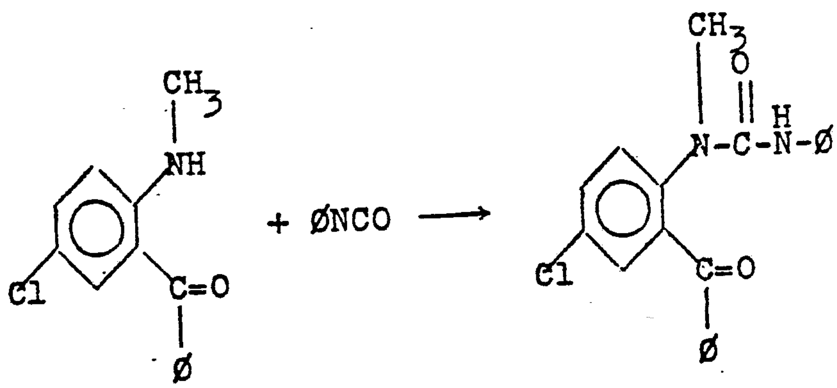


A solution of 12.3 g (0.05 mole) of 5-chloro-2-methylaminobenzophenone and 11.9 g (0.1 mole) of phenyl isocyanate in 50 ml of methylene chloride was refluxed for 3 days, and then evaporated to dryness under reduced pressure. The residual syrup was stirred with ether until it crystallized. The crystals were collected on

a filter and washed with ether to give 12.06 g (66%) of 1-(2-benzoyl-4-chlorophenyl)-1-methyl-3-phenylurea as light yellow crystals. A sample was recrystallized from ethanol to give colorless crystals: m.p. 158-160°; $^1$H nmr (DMSO-$d_6$) δ 3.41 ppm (s, 3H), 6.7-7.5 ppm (m, 13H).

Anal. Calc'd. for $C_{21}H_{17}ClN_2O_2$: C, 69.13; H, 4.70; N, 7.68

Found: C, 68.82; H, 4.73; N, 7.48

Part B. 6-Chloro-1-methyl-4-phenyl-2(1H)quinazolinone 3-Oxide

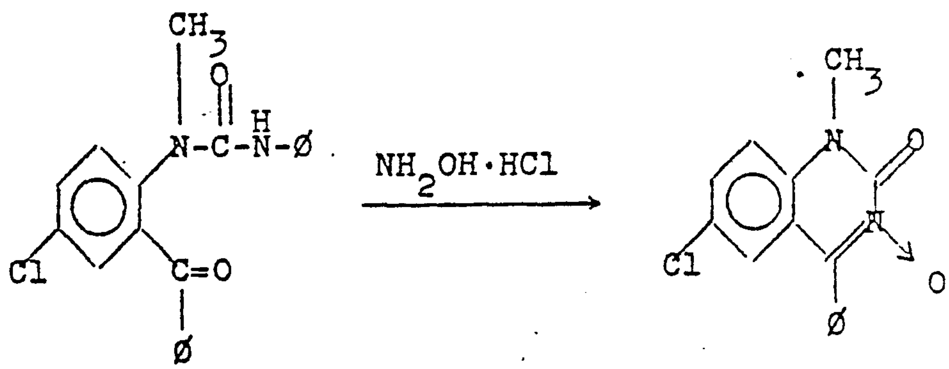


A mixture of 3.65 g (0.01 mole) of 1-(2-benzoyl-4-chlorophenyl)-1-methyl-3-phenylurea and 2.09 g (0.03 mole) of hydroxylamine hydrochloride in 50 ml of ethanol was stirred and refluxed for 5 days. The reaction mixture was cooled, and the suspended solid was collected on a filter, washed with alcohol, and dried in air to give 1.80 g (63%) of 6-chloro-1-methyl-4-phenyl-2(1H)quinazolinone as yellow crystals, m.p. 289-291°.

EXAMPLE 7

Part A. 6-Chloro-4-ethoxy-3,4-dihydro-3,4-diphenyl-2(1H)-quinazolinone

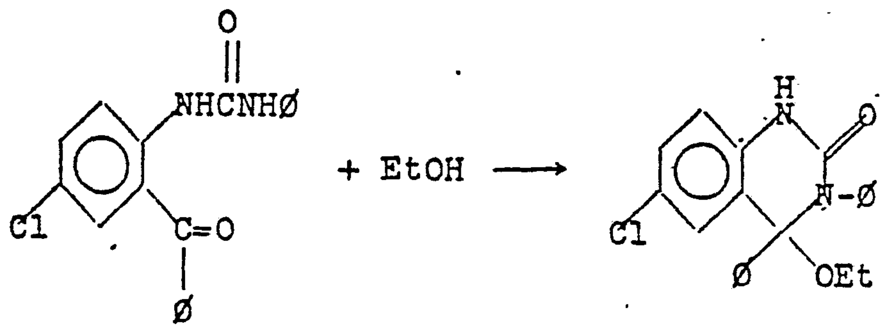


A solution of 10.0 g (0.285 mole) of 1-(2-benzoyl-4-chlorophenyl)-3-phenylruea in 50 ml ethanol was refluxed for 18 hours, and then cooled. The solid that formed was collected on a filter and washed with ethanol to give 9.46 g (88%) of 6-chloro-4-ethoxy-3,4-dihydro-3,4-diphenyl-2(1H)quinazolinone as colorless crystals: m.p. 209-211°. The $^1H$ nmr spectrum shows the presence of an ethyl group in addition to aromatic hydrogens.

<u>Anal</u>. Calc'd. for $C_{22}H_{19}ClN_2O_2$: C, 69.74; H, 5.05; N, 7.40

Found: C, 70.12; H, 5.05; N, 7.35

<u>Part B. 6-Chloro-4-phenyl-2(1H)quinazolinone 3-Oxide</u>

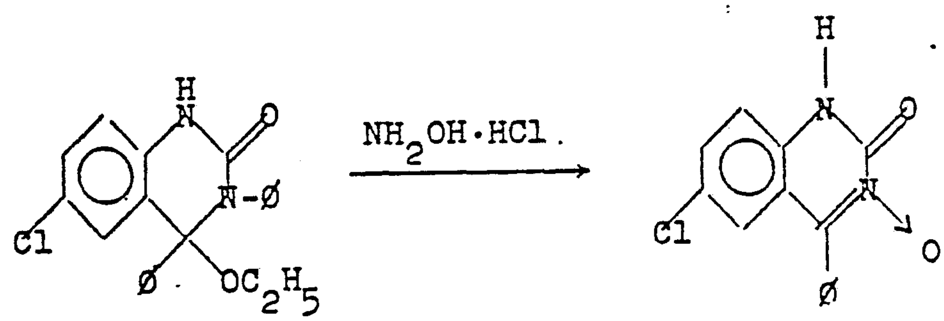


A mixture of 3.51 g (0.0093 mole) of 6-chloro-4-ethoxy-3,4-dihydro-3,4-diphenyl-2(1H)quinazolinone and 2.09 g (0.03 mole) of hydroxylamine hydrochloride in 50 ml alcohol was refluxed for 4 days, and then cooled. The solid that formed was collected on a filter, washed with ethanol, and dried in air to give 1.82 g (72%) of 6-chloro-4-phenyl-2(1H)quinazolinone 3-oxide as yellow crystals, m.p. 267-269°.

EXAMPLE 8

6-Chloro-4-phenyl-2(1H)quinazolinone 3-Oxide

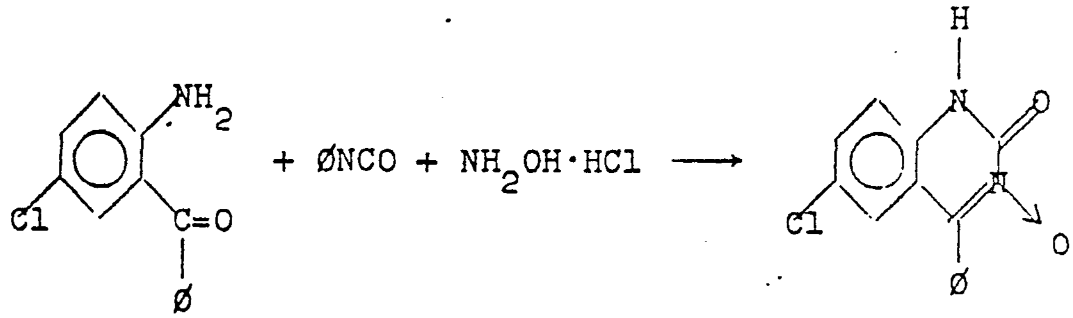


A mixture of 12.6 g (0.05 mole) of 2-amino-5-chlorobenzophenone and 6.55 g (0.055 mole) of phenyl isocyanate was heated on a steam-bath for 30 minutes, and then 250 ml ethanol and 10.43 g (0.15 mole) of hydroxylamine hydrochloride were added and the mixture was refluxed for 2 days and then cooled. The solid precipitate that formed was collected on a filter, washed with ethanol, and dried in air to give 9.42 g (69%) of 6-chloro-4-phenyl-2(1H)quinazolinone 3-oxide as yellow crystals, m.p. 267-269°.

EXAMPLE 9

6-Chloro-4-phenyl-2(1H)quinazolinone 3-Oxide

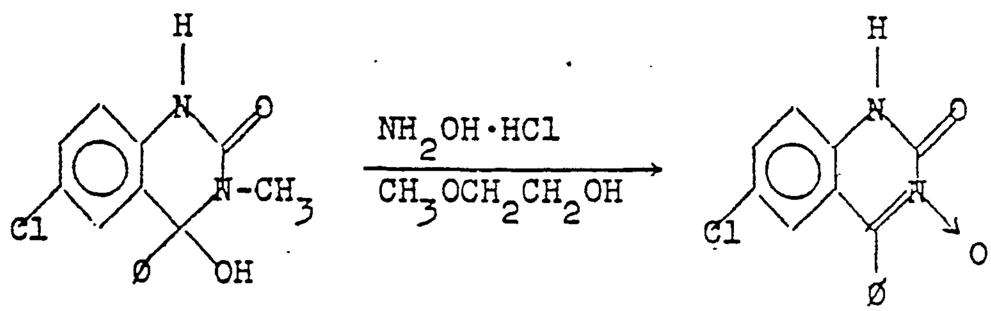


A stirred mixture of 2.89 g (0.01 mole) of 6-chloro-3,4-dihydro-4-hydroxy-3-methyl-4-phenyl-2(1H)-quinazolinone and 2.09 g (0.03 mole) of hydroxylamine hydrochloride in 50 ml of 2-methoxyethanol (ethylene

glycol monoethyl ether) was refluxed for 2 hours, and then cooled to 0°. The solid that formed was collected on a filter, washed with ethanol, and dried in air to give 1.40 g (51%) of 6-chloro-4-phenyl-2(1H)quinazolinone 3-oxide as yellow crystals, m.p. 267-269°.

EXAMPLE 10

Part A. 6-Bromo-3,4-dihydro-4-hydroxy-3-methyl-4-phenyl-2(1H)quinazolinone

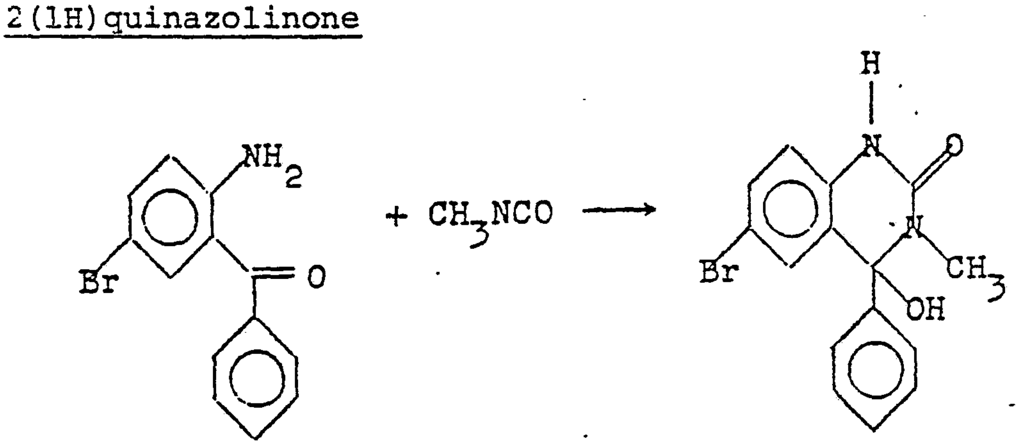

A solution of 14.70 g (.053 mole) of 2-amino-5-bromobenzophenone and 6.0 g (0.21 mol) of methyl isocyanate in 75 ml of methylene chloride was refluxed for two days and then cooled. The solid portion of the reaction mixture was collected on a filter and washed with methylene chloride to give 16.18 g (90% yield) of 6-bromo-3,4-dihydro-4-hydroxy-3-methyl-4-phenyl-2(1H)-quinazolinone as a white crystalline powder: m.p. 293-294° (dec.).

$^1H$ nmr (DMSO-$d_6$) δ 2.66 ppm (s, 3H), 6.5-7.5 ppm (m, 8H).

Anal. Calc'd. for $C_{15}H_{13}BrN_2O_2$: C, 54.07; H, 3.93 N, 8.41

Found: C, 54.24; H, 3.89; N, 8.12

Part B. 6-Bromo-4-phenyl-2(1H)quinazolinone 3-Oxide

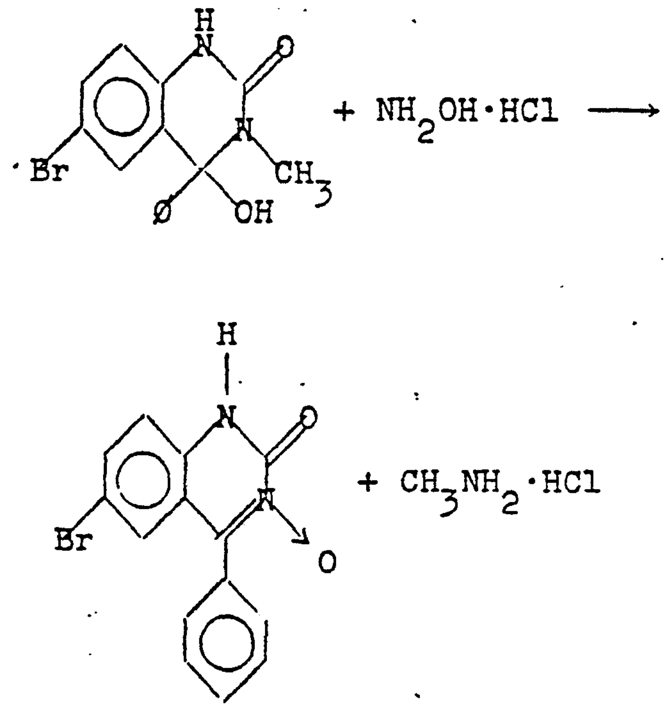


A stirred mixture of 28.28 g (.085 mol) of 6-bromo-3,4-dihydro-4-hydroxy-3-methyl-4-phenyl-2(1H)-quinazolinone and 17.6 g (.25 mol) of hydroxylamine hydrochloride in 425 ml of ethanol was refluxed for 192 hours and then cooled. The solid portion of the reaction mixture was collected on a filter, washed with ethanol, and dried in air to give 20.92 g (.066 mol, 78%) of 6-bromo-4-phenyl-2(1H)quinazolinone 3-oxide as light yellow crystals: m.p. 275-276°.

EXAMPLE 11

Part A. 6-Chloro-3-ethyl-4-(2-fluorophenyl)-3,4-dihydro-4-hydroxy-2(1H)quinazolinone

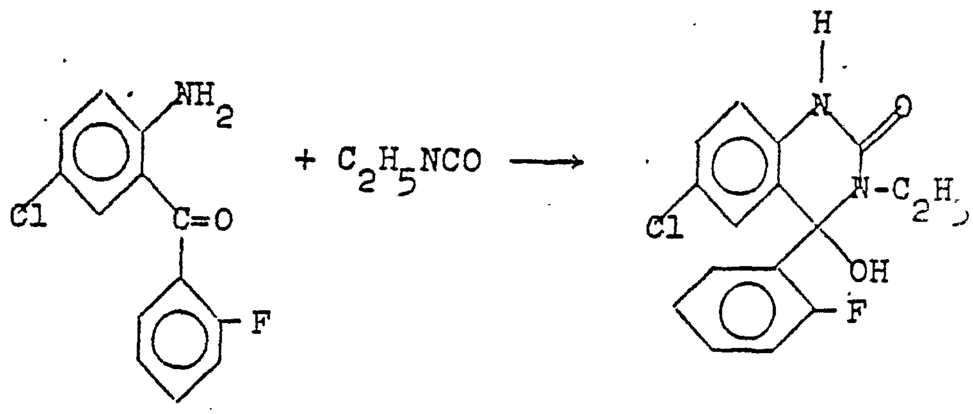


A mixture of 25 g (0.1 mole) of 2-amino-5-chloro-2'-fluorobenzophenone and 35.5 g (0.5 mole) of ethyl isocyanate was refluxed for 20 hours, and then cooled. The solid portion of the reaction mixture was collected on a filter and washed with methylene chloride to give 19.6 g (61%) of 6-chloro-3-ethyl-4-(2-fluorophenyl)-3,4-dihydro-4-hydroxy-2(1H)quinazoline as a white crystalline powder: m.p. 176-178° (dec.); $^{19}F$ nmr (DMSO-$d_6$) -114.0 ppm; ir (KBr) at 6.24 μ for C=O.

Anal. Calc'd. for $C_{16}H_{14}ClFN_2O_2$: C, 59.92; H, 4.40; N, 8.73

Found: C, 60.11; H, 4.44; N, 8.83

Part B. 6-Chloro-4-(2-fluorophenyl)-2(1H)quinazolinone 3-Oxide

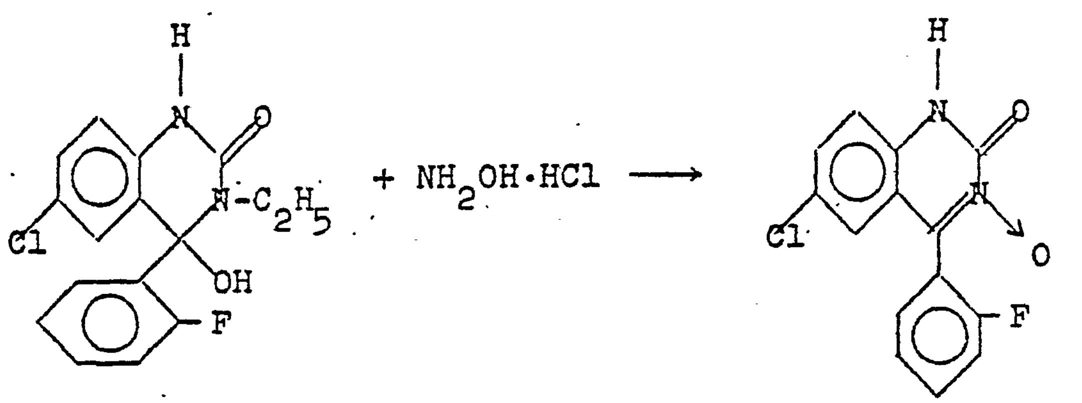

A stirred mixture of 18.0 g (0.056 mole) of 6-chloro-3-ethyl-4-(2-fluorophenyl)-3,4-dihydro-4-hydroxy-2(1H)quinazoline, 11.8 g (0.17 mole) of hydroxylamine hydrochloride, and 280 ml of ethanol was refluxed for 3 days. The reaction mixture was cooled, and the precipitate was collected on a filter and washed with ethanol to give 6.67 g (47%) of 6-chloro-4-(2-fluorophenyl)-2(1H)quinazoline 3-oxide as a yellow crystalline powder; m.p. 268-270° (dec.); $^{19}F$ nmr (DMSO-$d_6$) δ 111.1 ppm.

Anal. Calc'd. for $C_{14}H_8ClFN_2O_2$: C, 57.85; H, 2.77; N, 9.64

Found: C, 58.01; H, 2.83; N, 9.59

Table I shows additional ureas and hydroxyquinazolinones which can be prepared by the process disclosed and exemplified above using the appropriate aminobenzophenone and a suitable organic isocyanate.

TABLE I

Preparation of Substituted Ureas and Hydroxyquinazolinones

| Aminobenzophenone | Organic Isocyanate | Product |
|---|---|---|

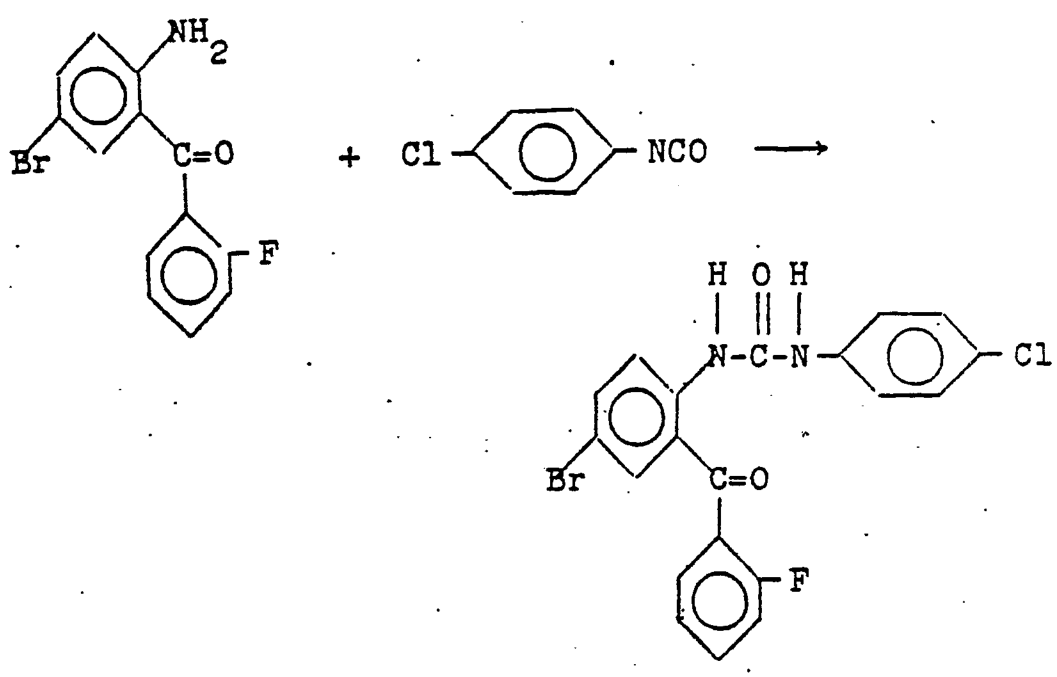

| Aminobenzophenone | Organic Isocyanate | Product |
|---|---|---|

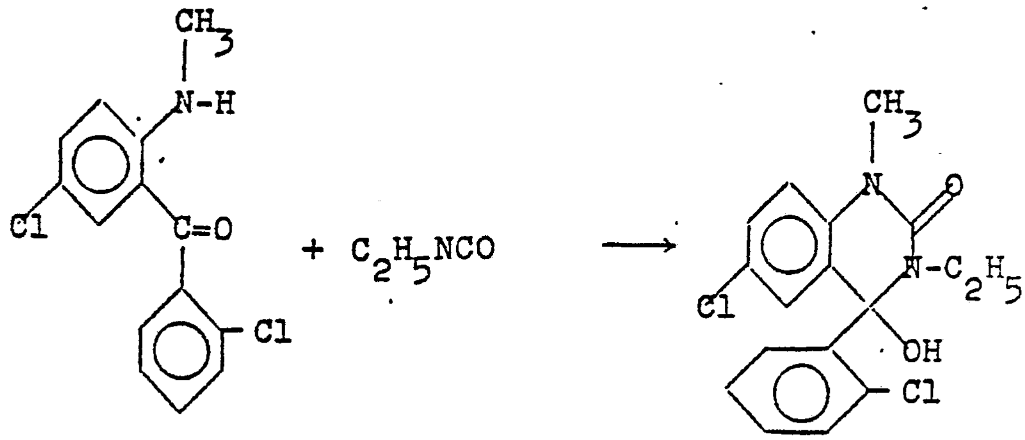

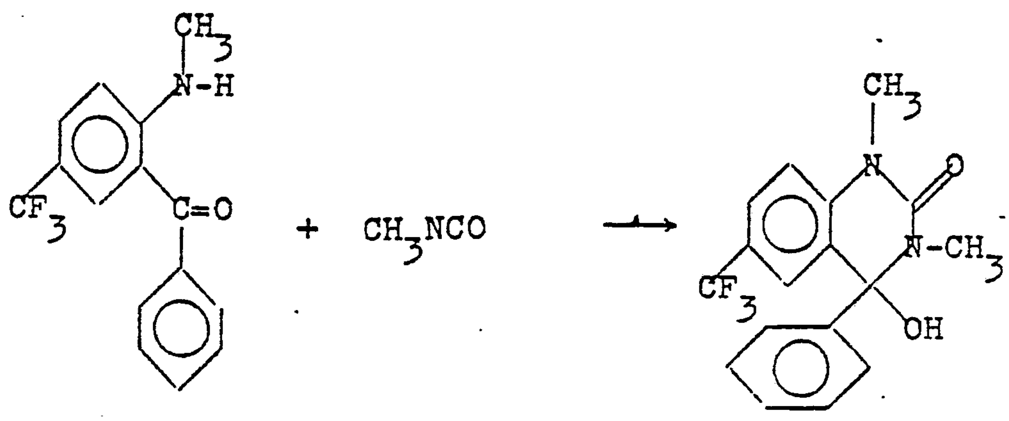

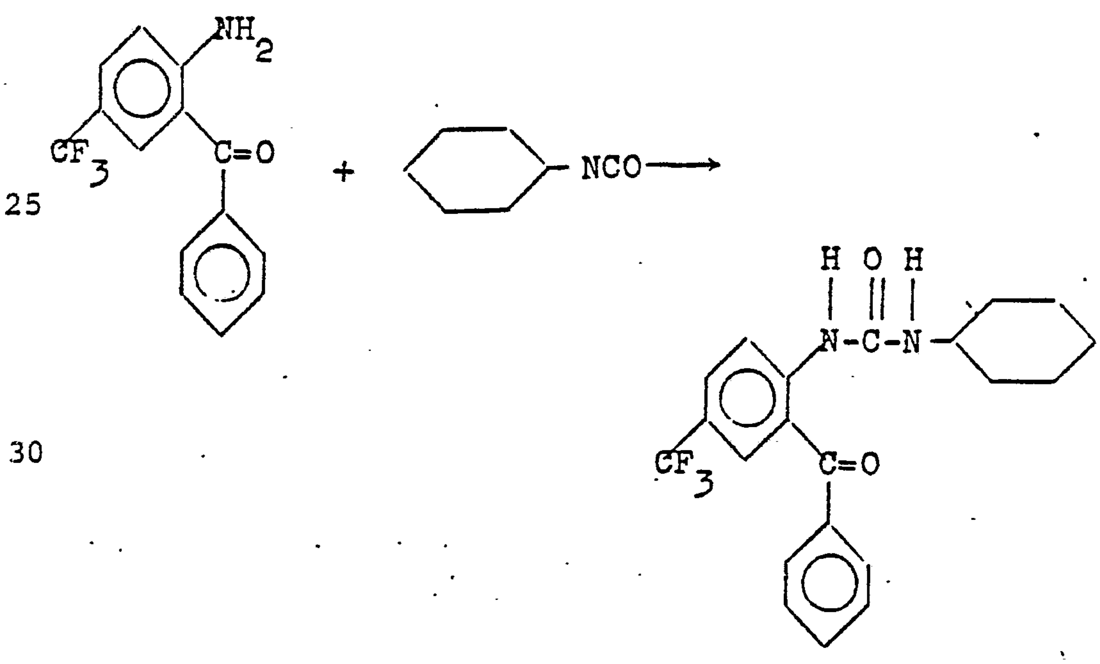

Table II shows additional quinazolinone oxides which can be prepared by the process disclosed and exemplified above using the appropriate isocyanate adduct and hydroxylamine or a suitable salt thereof.

TABLE II

Preparation of Selected Quinazolinone Oxides

| Isocyanate Adduct | | Hydroxylamine | Quinazolinone Oxide |
|---|---|---|---|
| 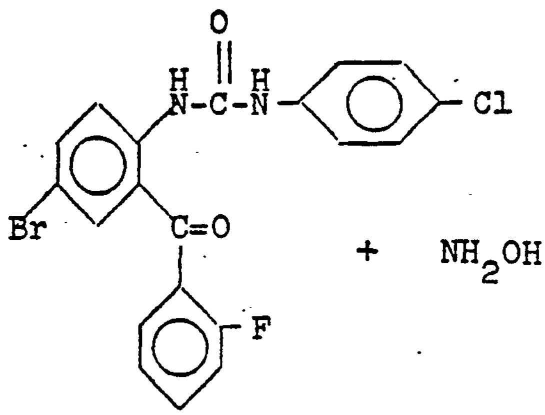 | + | $NH_2OH \cdot HCl$ | 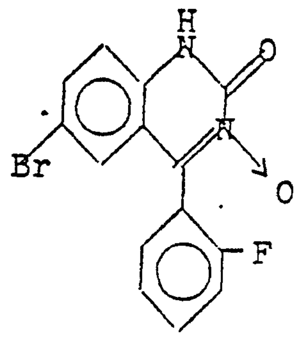 |
| 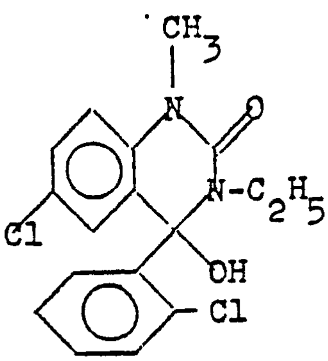 | + | $(NH_3OH)_2SO_4$ | 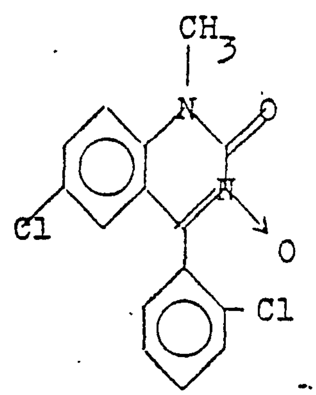 |
| 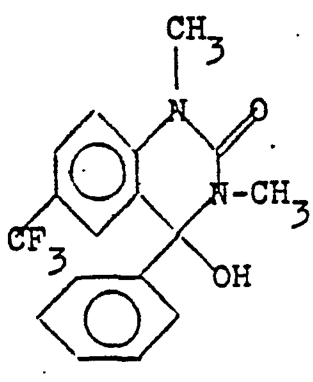 | + | $NH_2OH$ | 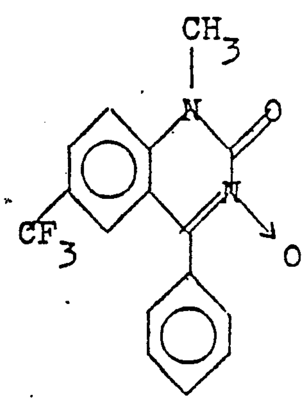 |

TABLE II (cont'd.)

| Isocyanate Adduct | Hydroxylamine | Quinazolinone Oxide |
| --- | --- | --- |

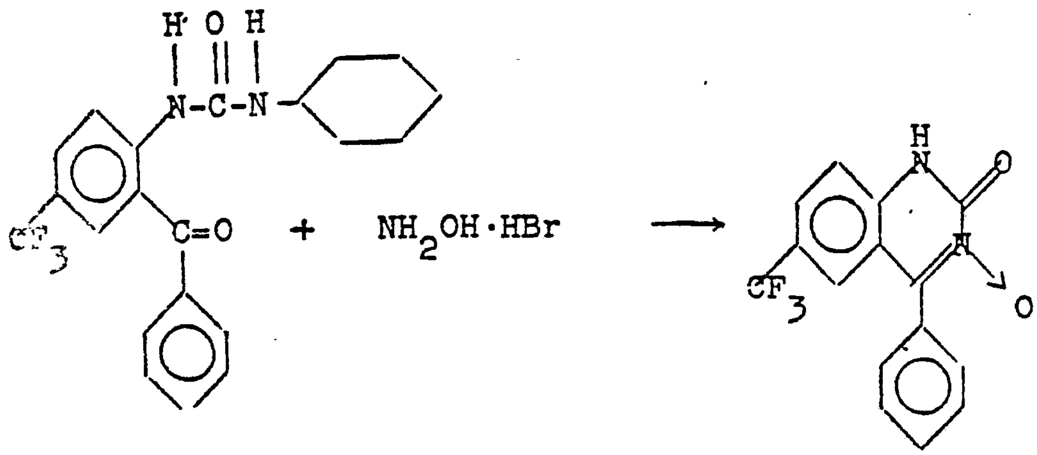

Patentanwälte
Dr.-Ing. Schönwald Dr.-Ing. Th. Meyer Dr.-Ing. Eishold
Dr. Fues · Dipl.-Chem. Alek von Kreisler
Dipl.-Chem. Carola Keller · Dipl.-Ing. Selting
5 Köln 1, Deichmannhaus

0000149



What is claimed is:

1. A process for preparing a compound of the formula:

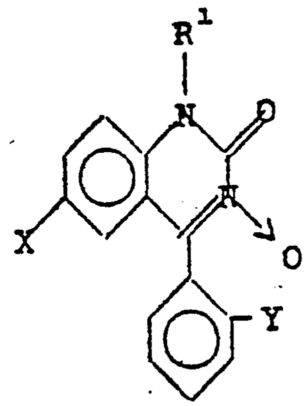

where

X is Cl, Br, $NO_2$ or $CF_3$;

Y is H, Br, Cl, or F; and

$R^1$ is H or $CH_3$;

which comprises treating either or both of compounds of the formulae:

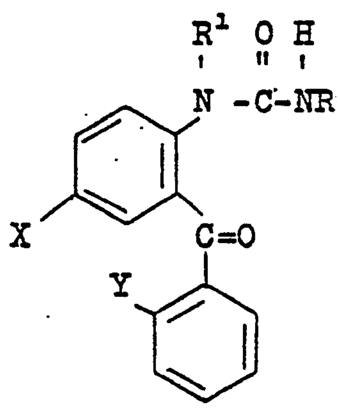

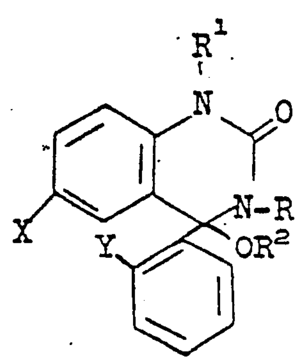

0000149

with an acid salt of hydroxylamine, where R is hydrocarbyl or halohydrocarbyl of up to 8 carbon atoms, and

$R^2$ is H or alkyl of 1-6 carbon atoms.

2. A process for preparing a compound of the formula:

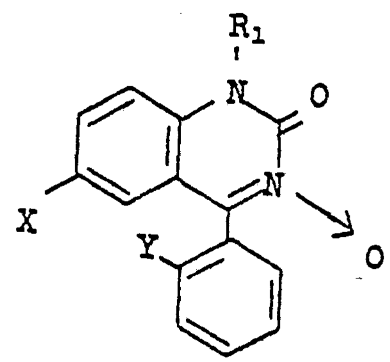

where

X is Cl, Br, $NO_2$ or $CF_3$;

Y is H, Br, Cl or F; and

$R^1$ is H or $CH_3$;

which comprises the following steps in sequence:

(a) reacting a compound of the formula:

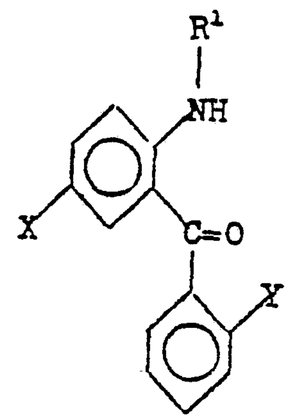

with an organic isocyanate of the formula RNCO to produce a compound or compounds of one or both of the following formulae:

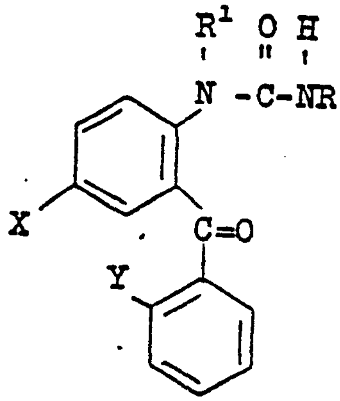

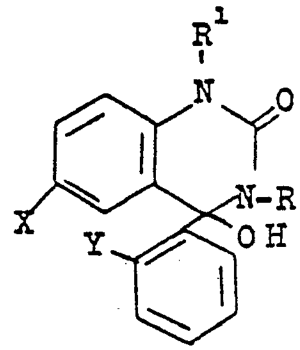

(b) treating the reaction product or products of step (a) with an acid salt of hydroxylamine, where R is hydrocarbyl or halohydrocarbyl of 1-8 carbon atoms.

3. A process for preparing a compound of the formula:

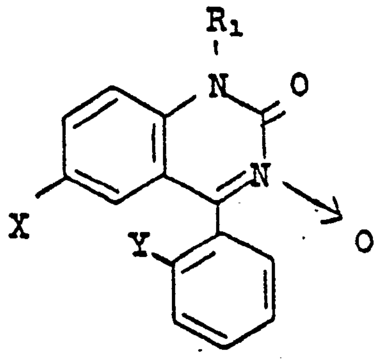

where

X is Cl, Br, $NO_2$ or $CF_3$;

Y is H, Br, Cl or F; and

$R^1$ is H or $CH_3$;

which comprises the following steps in sequence:

(a) reacting a compound of the formula:

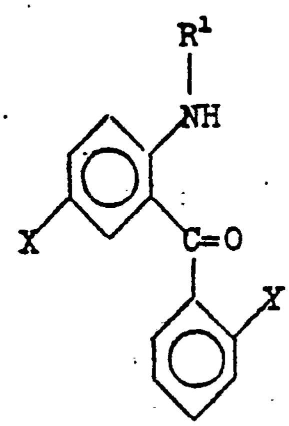

with an organic isocyanate of the formula RNCO to produce a compound of the formula:

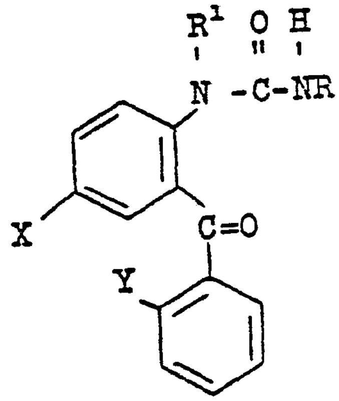

(b) heating the reaction product of step (a) in a lower aliphatic alcohol of the formula $R^2OH$ to produce a compound of the formula:

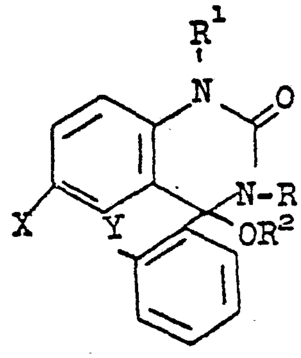

(c) treating the reaction product of step (b) with a suitable acid addition of hydroxylamine, where $R^2$ is alkyl of 1-6 carbon atoms.

European Patent Office

# EUROPEAN SEARCH REPORT

0000149

Application number
EP 78 10 0163

## DOCUMENTS CONSIDERED TO BE RELEVANT

| Category | Citation of document with indication, where appropriate, of relevant passages | Relevant to claim |
| --- | --- | --- |
| | CH - A - 573 412 (SUMITOMO)<br>* Column 1 to 6 *<br>---- | 1-3 |

CLASSIFICATION OF THE APPLICATION (Int. Cl.²)

C 07 D 239/82

TECHNICAL FIELDS SEARCHED (Int.Cl.²)

C 07 D 239/82

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
| --- | --- | --- |
| The Hague | 14-09-1978 | FRANCOIS |